(19) Europäisches Patentamt / European Patent Office / Office européen des brevets

(11) **EP 4 186 562 B1**

(12) **EUROPEAN PATENT SPECIFICATION**

(45) Date of publication and mention
of the grant of the patent:
**29.11.2023 Bulletin 2023/48**

(21) Application number: **21210650.4**

(22) Date of filing: **26.11.2021**

(51) International Patent Classification (IPC):
**A61N 5/10** $^{(2006.01)}$

(52) Cooperative Patent Classification (CPC):
**A61N 5/1001; A61N 5/1071; A61N 5/1075;**
A61N 2005/1076

(54) **SYSTEM AND METHOD FOR BRACHYTHERAPY PROCEDURE PLANNING AND VERIFICATION**

SYSTEM UND VERFAHREN ZUR PLANUNG UND VERIFIZIERUNG EINER
BRACHYTHERAPIEPROZEDUR

SYSTÈME ET PROCÉDÉ DE PLANIFICATION ET DE VÉRIFICATION DE PROCÉDURE DE
CURIETHÉRAPIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**31.05.2023 Bulletin 2023/22**

(73) Proprietors:
• **Vilnius University**
**01513 Vilnius (LT)**
• **National Cancer Institute**
**08660 Vilnius (LT)**

(72) Inventors:
• **Gaubas, Eugenijus**
**06203 Vilnius (LT)**
• **Ceponis, Tomas**
**04116 Vilnius (LT)**
• **Pukas, Kornelijus**
**09205 Vilnius (LT)**
• **Rumbauskas, Vytautas**
**10230 Vilnius (LT)**
• **Uzgiryte, Milita**
**65167 Varena (LT)**
• **Venius, Jonas**
**08453 Vilnius (LT)**
• **Akelaitis, Kestutis**
**12124 Vilnius (LT)**
• **Cicinas, Aleksandras**
**09305 Vilnius (LT)**

(74) Representative: **Pakeniene, Ausra**
**AAA Law**
**A. Gostauto street 40B**
**03163 Vilnius (LT)**

(56) References cited:
WO-A1-2021/116756    WO-A2-2015/145300
WO-A2-2020/246728    KR-A- 20200 134 417
US-A1- 2014 350 325

**Description**

FIELD OF INVENTION

This invention relates to medical equipment and methods of use thereof. More specifically, it discloses a method of simultaneous and self-consistent positioning and dosimetry control of brachytherapy radionuclide emitters for brachytherapy procedure planning and verification, before and for the brachytherapy treatment, and a corresponding system and phantom apparatus implementing this method.

BACKGROUND ART

The brachytherapy planning and treatment procedures comprise an inevitable and specific component of the identification and reconstruction of the radiation emitters within catheters and applicators used in dynamic treatment [1], when the actual position of radiation emitters should be firstly reconstructed in the phantom-medium, and only then in the patient's body. Brachytherapy planning can be implemented using 2D representations and documentations for simplified brachytherapy applications or using 3D-imaging techniques such as computer tomography (CT), magnetic resonance (MR), and ultrasound scanning (US) for more complex and interactively administered brachytherapy treatments.

Reconstruction of the radiation emitters within catheters requires the time-resolved control of the radioactive source position [2]. The time-resolved dosimetry is more sensitive in the administration of treatment deviations [3]. Moreover, the instantaneous dose rate measurements and accumulated dose maps are necessary for brachytherapy planning and brachytherapy *in vivo* treatment procedures. The precise localization of the radionuclide emitters (5) and dosimeters (4) in high-dose-gradient fields is crucial for adequate evaluations of measurement uncertainty. As usual, the different apparatuses are employed for identification and control of the mutual positions of a patient and the radiation emitters. Qualitatively, the methods for control and imaging of the instantaneous localization of medical probes and patient anatomy can be classified as 1) optical tracking, based on video-cameras and visual markers [3], 2) the electromagnetic (EM) tracking, based on detection of magnetic fields due to the induced currents in solenoids or fluxgate sensors [4, 5], 3) the X-ray based flat-panel imagers or computer tomography (CT) based 3D imaging systems, and 4) the combined positioning systems composed of dosimeter-sensors acting as position and dose detectors [3,6].

Optical tracking is mainly employed in surgery procedures when visual scenes can be labelled and directly controlled. However, special techniques, such as Brachy-View [3], based on pinhole $\gamma$-camera, where a linear array of pinholes in collimator is combined with an array of pixelated Si detectors, can be implemented for real-time tracking of radiation emitters by analysing multiple images projected onto the detector array. The emitter location is there evaluated by finding the space point with minimum perpendicular distance to all the back-projected lines through the corresponding pinholes. However, modelling of the pinhole geometry and Monte-Carlo simulations should be performed to reach adequate sensitivity and spatial resolution without radiation overexposing healthy organs. The catheters and needle-type probes are tracked inside the human body, and the electromagnetic (EM) tracking means have emerged as the method for localization of small EM sensors in a given EM field [5]. The EM tracking phenomenon arises from the fact that electromagnets are responsible for producing alternating (ac) or quasi-static (dc) magnetic fields, which induce currents in solenoid sensors, embedded in the detectors. The phenomenon responsible for the operation of these tracking systems relies solely on magnetic induction. EM tracking accuracy can be compromised by magnetic field distortion due to nearby medical diagnostic devices or ferromagnetic objects. Furthermore, additional hardware components of the tracking system, such as the EM field generator, must be placed close to the patient and imaging devices equipped with fragile EM sensors. The X-ray imaging systems are appropriate for when large volumes of the patient's body are tracked as in external radiation treatment using electron or X-ray beams. However, the usage of X-rays for a patient's positioning can be detrimental, especially in brachytherapy when rather small ranges should be imaged and controlled, while rather intensive irradiations are delivered to the patient for auxiliary positioning purposes. The more intricate emitter tracking systems, based on the so-called Magic Phantom [3], can be designed where it is possible to measure the delivered irradiation dose with simultaneous source position and timing accuracy by using the high timing resolution of the analogical front-end electronics.

Examples of positioning and dosimetry methods and instruments for the control of the brachytherapy radiation emitters in brachytherapy planning and treatment procedures are described in research papers [1-6].

Patent documents considered as the closest ones to the present invention are these:

1) A method and apparatus based on "A phantom for verification of the accuracy of HDR brachytherapy planning and Phantom device having the phantom", described in the Korean Patent KR100613244B1, discloses a high-dose-rate brachytherapy phantom for quality control of a treatment planning computer that establishes a treatment plan for brachytherapy. The phantom for brachytherapy is composed of an acrylic assembly comprising a rectangular parallelepiped holder, to which an applicator is fixed, and an upper, lower block, and side block to which the holder is inserted and fixed, and various dose measurements and distribution measurements are possible by emulating the human body by this phantom. However, the disclosed high-dose-rate brachytherapy phantom has a complex structure and can be applied only to a specific type of dose-measuring device, as suggested in this patent document, however, the other existing dose-measuring devices are not applicable herein.

2) Another method and apparatus based on "Phantom device for measuring proximity radiation dose (Phantom apparatus for measuring the dose of brachytherapy radiation)", described in the Korean patent KR101752972B1, discloses a phantom device for measuring proximity radiation dose, where the phantom and method are capable of improving treatment precision through accurate prediction of radiation dose irradiated to a patient during the brachytherapy treatment. This phantom device for measuring proximity radiation dose (to achieve the above object) includes a base-plate provided with 1) a plurality of installation grooves in which 2) a plurality of dosimeters are installed, and 3) a cover for covering the base-plate with a plurality of cover grooves covering the plurality of dosimeters and a 4) photosensitive plate stacked on the base-plate with the cover plate interposed there between, and in which 5) a radiation photoreceptor is embedded. The dosimeter includes at least one of a glass dosimeter, a MOSFET (Metal Oxide Field Effect Transistor) dosimeter, an OSLD (Optically Stimulated Luminescence Dosimeter) dosimeter, and a Thermo-Luminescence Dosimeter (TLD) dosimeter. The base-plate, the cover plate, and the photosensitive plate are formed of a synthetic resin material including acrylic. The photosensitive plate is provided with grid-shaped coordinates for measuring radiation dose distribution. A fluorescent material is provided in a plurality of positions on at least one of the base-plate, the cover plate, and the photosensitive plate. The base-plate, the cover plate, and the photosensitive plate are fixed to each other by a plurality of fixing clips that are coupled in a stacked state.

3) The closest prototype patent to the present invention is considered to be the Australian patent application AU2008100728A4 which discloses "A real-time treatment dose verification system for afterloader brachytherapy systems". This system measures the brachytherapy treatment radiation dose and the geometrical distribution of the radiation dose during the treatment delivery by a brachytherapy afterloading system. The system can measure the position of the radiation isotope source (emitter) as it moves through each catheter, in real-time over the duration of the entire brachytherapy treatment. It provides a comparison of the amount of radiation dose being delivered as well as the geometric distribution of the dose, against the planned treatment dose and distribution. The system consists of a two-dimensional flat panel imaging device that is positioned against the patient during the treatment. The flat panel imager is then linked to a control computer system which reads the data from the imager and compares the radiation measured to the planned patient treatment. The flat panel imager records the patient exit radiation dose at a known geometrical plane from the brachytherapy implant, as a digital image. The flat panel imager is responsive to the radiation X-ray energies of the radioactive isotope. For example, a common radiation isotope source is Iridium-192, which has mean X-ray energy of 390 keV. The imager will also be responsive at X-ray energies of 120 keV to 140 keV. The geometrical location of the flat panel imager in relation to the patient is determined using an external kilovoltage X-ray system. The flat panel imager is positioned for treatment, then a kilovoltage image is taken through the patient, to record the flat panel imager and patient relationship. This image is supplied to the processing computer before brachytherapy treatment commences, and the geometrical position is calculated. As the treatment delivery proceeds, with the afterloader stepping the radioactive isotope source through each catheter, the image recorded by the flat panel imager is transferred to the control computer system where the measured image is subtracted from a planned image. All measured and planed image comparisons will be made progressively as treatment is delivered at some predetermined time interval. An error threshold is set, producing an error flag when the delivered dose differs by more than a critical amount from the planned dose. This error flag will notify the brachytherapy treatment operator to stop the treatment. The data collected throughout the treatment will also provide a total delivered radiation dose record. The dose recorded by the flat panel imager is the patient exit dose. The process computer, as well as comparing planned and delivered exit dose, will also back project the exit dose through the patient, to the implant site, and provide measured radiation dose at any plane within the tumour site. The back-projection method, therefore, provides a two-dimension in-vivo dose verification system without any invasive procedure. This process can be applied to any part of the human body.

The closest patent document WO2020246728 discloses a dose measurement device for brachytherapy, comprising: a radiation emission unit provided on the inner side of at least one from among tubular body units so as to emit radiation; and a dose measurement unit for measuring, at a plurality of points, a dose of the radiation emitted from the radiation emission unit, wherein a dose of radiation emitted in proximity to a lesion of a patient and near healthy organs can be measured in real time. For example, WO2020246728 cites several paragraphs disclosing, that *"the insertion unit is at least partially inserted into the body, the body unit includes a first body unit to a third body unit, the second body unit and the third body on both sides of the first body unit",* also presented in WO2020246728 Fig. 3.

The above-reviewed brachytherapy methods, systems, and apparatuses have a lack of efficient instrumentation (further disclosed by the present invention) for precise positioning of the radionuclide emitters and additional X-ray sources (such as Computer Tomography imagers) while those inaccurately exposed can be detrimental for a patient. An efficient and precise operation of instrumentation would require several bases and florescent plates with a lot of dosimetry grooves where the solution is a complex optimization task requiring long exposures of X-ray sources, to estimate simultaneously 3D-positions of these X-ray sources and the accumulated 3D-irradiation dose.

SUMMARY OF INVENTION

The present invention is defined by the claims. All the methods described below, which are performed during patient's treatment, do not form part of the invention.

This invention discloses:

- a system comprising a synchronous 3D-scanner tightly linked to a base-plate, and further, comprising a brachytherapy phantom apparatus;
- a self-consistent method for navigating the brachytherapy radionuclide emitters, as well as, estimating the spatially cumulated irradiation dose 3D-map, using the aforementioned system being coupled either

  - to the phantom for brachytherapy procedure planning and verification,
  - or to the patient's body for a brachytherapy treatment procedure.

In this system, the base-plate contains a mesh-work with a grid-shaped 2D-coordinate-scale indicators, and with a plurality of the catheter-installation grooves within this coordinate-scale and mesh-work. Further, the base-plate with its 2D-coordinate scale indicators and the plurality of grooves is tightly spatially linked by 3D-coordinates to the brachytherapy phantom or patient's body, by using one or more labels marked on the brachytherapy phantom or patient's body. Furthermore, the invariability of this spatial linking via labels, during the brachytherapy procedure period, can be supported using remote-position-verification-devices (RPVD), such as LiDAR, ultrasound, or laser distance meters, installed within the base-plate.

The self-consistent method, together with this system, is intended for (i) instantaneous (real-time) 3D-positioning of brachytherapy radionuclide emitters and (ii) simultaneous spatial 3D-dosimetry estimation. The precise (i) 3D-positioning and then (ii) spatial dosimetry tasks are inevitable during the brachytherapy procedure planning and verification stage, and subsequently, during the brachytherapy *in vivo* treatment stage. The operation of the method and system is based on the triangulation technique using needle-type catheter probes with radiation sensors inside (at least, 3 such catheter probes) tightly fixed in the base-plate, within time-space-resolved 3D-coordinates, thus comprising a 3D-scanner. When being coupled to the brachytherapy phantom, this system and method are also linked to a plurality of probe-installation grooves in the phantom, those matching the catheter(probe)-installation grooves in the base-plate. At least three needle-type catheters with probes comprise a 3D-scanner with the spatial arrangement of internal scintillator-sensors. The sensors operate as optical beacons and dosimetry diodes installed within the needle-type scaling catheters with those to be precisely 3D-positioned within either:

- the brachytherapy phantom apparatus, for brachytherapy procedure planning and verification,
- or a therapeutic patient's tumour site, the boundary contour of which is simultaneously imaged, preferably in real-time, by routine means of computer tomography (CT) or ultrasound scanning (US), during the brachytherapy treatment stage.

The operation of the 3D-scanner using the scintillator-sensors is based on scintillator signals, induced by the same brachytherapy radionuclide emitter. The induced scintillation signals are transferred by fiberscopes to optical-electrical-digital converters, where the converted digital data is transmitted to the controlling computer for further processing. The scintillation signals can be also employed for positioning the computer tomography (CT) X-ray source, and for linking the CT X-ray source coordinates to the base-plate position and scale, to link the 3D-scanner coordinates to the tumour site and its boundary contour.

The 3D-scanner is combined with radionuclide dosimeters, operating through electrical signalling and made as the semiconductor radiation sensors, installed within the same needle-type catheters, together with the scintillating sensors. Triangulation of the signals from the radiation sensors is combined with the triangulation performed by scintillation sensors, to reach the enhanced precision in 3D-positioning of the radionuclide emitters in time-space-resolved coordinates.

This 3D-scanner system, together with the phantom and with the spatial positioning and dosimetry methods efficiently facilitates the brachytherapy procedure during its planning and verification stage. Subsequently, the obtained verification results can be effectively used for the brachytherapy *in vivo* treatment stage.

DESCRIPTION OF DRAWINGS

The drawings are provided as a reference to possible embodiments and are not intended to limit the scope of the invention. Neither of the drawings nor the graphs presented herein should be construed as limiting the scope of the invention, but merely as an example of a possible embodiment.

**Fig. 1**   depicts a brachytherapy spatial positioning and dosimetry system - 3D-scanner (2) for brachytherapy procedure planning and verification, and the subsequent brachytherapy treatment procedure.

**Fig. 2.**   depicts a single probe (6) of the 3D-scanner (2); the probe is made of an insulating ribbon substrate (16), installed within a needle-type catheter (6); the probe has scintillating sensors (3) and dosimetry sensors (4) mounted on it; the sensors are connected to optical fibers (7) and electrical wires (17), respectively, for transferring sensor signals to optical/electrical/digital converters (18, 19).

**Fig. 3.**   depicts the system for brachytherapy positioning and dosimetry, arranged for brachytherapy procedure planning and verification, based on the 3D-scanner (2) and using a phantom (1), where the scintillator-sensor (3) signal, estimating 3D-position of a radiation source, is excited by:

•either a radionuclide emitter (5) inserted by an applicator (12) from a brachytherapy afterloader (13),

•or X-rays of the Computer Tomography imager (15).

as LiDAR (laser imaging, detection, and ranging), ultrasound, or laser distance meters, installed within the base-plate (10), for additional (dynamic) support of the bond by the label (11); **(b)** linking and matching different coordinate systems (3D-scales) of the base-plate (10), phantom (1), label (11) on the phantom (1) and patient's body, RPVD (22), tumour site in patient's body, and CT/US imager (15);

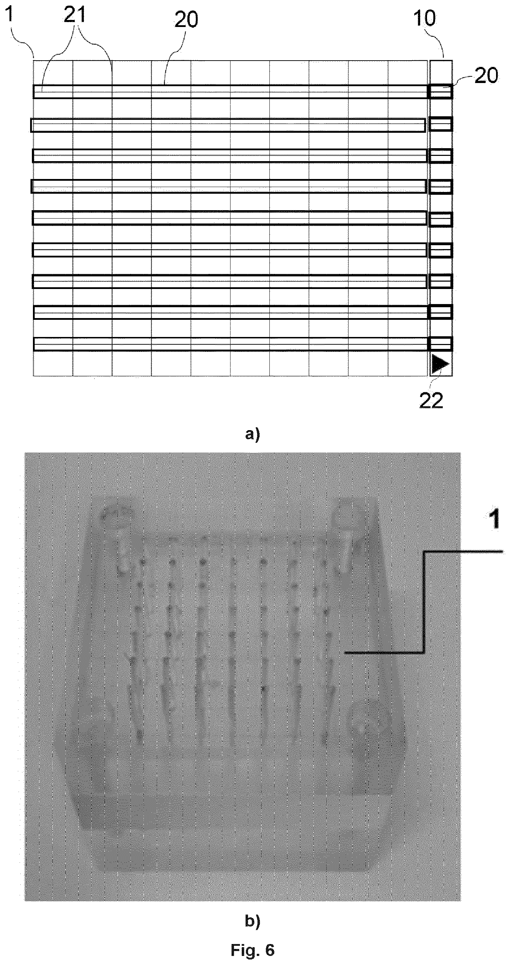

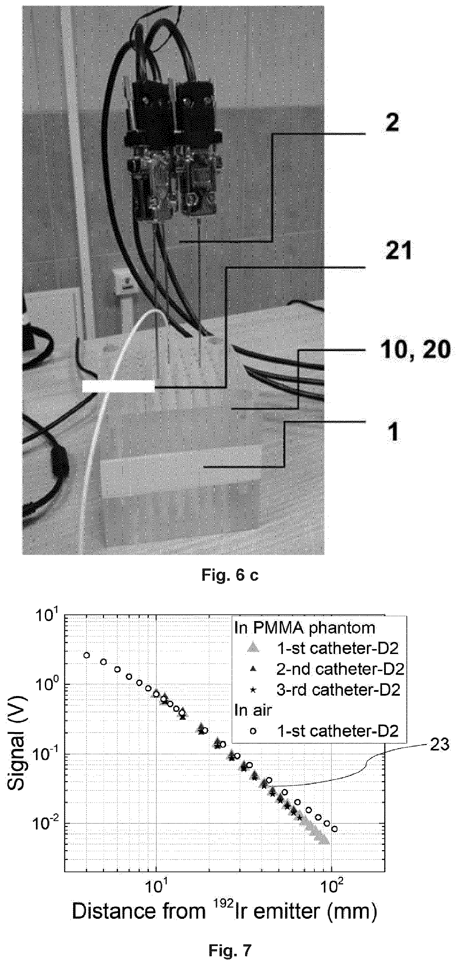

**Fig. 6.**   depicts **(a)** cross-section of cube-shaped phantom (1) for brachytherapy planning and verification; the phantom (1) comprises a mesh-work with X-Y-Z coordinates (21) and catheter-installation groves (20); the phantom (1) is attachable to the brachytherapy positioning and dosimetry system (2) through the base-plate (10) herewith matching to catheter installation grooves (20) of the base-plate (10); **(b)** an experimental embodiment of the phantom (1) and the base-plate (10), both made of transparent acryl; (c) an experimental setup of the 3D-scanner (3) with probes, the base-plate (10) and the phantom (1);

**Fig. 7.**   illustrates the characteristics (23) of 3D-scanner (2) calibration where the dosimetry sensor (4) signal is a function of distance from the $^{192}$Ir radionuclide emitter (5); the emitter (5) and dosimeter (4) allocated, correspondingly, in O- *and C*-catheters, where the catheters are installed in the phantom (1);

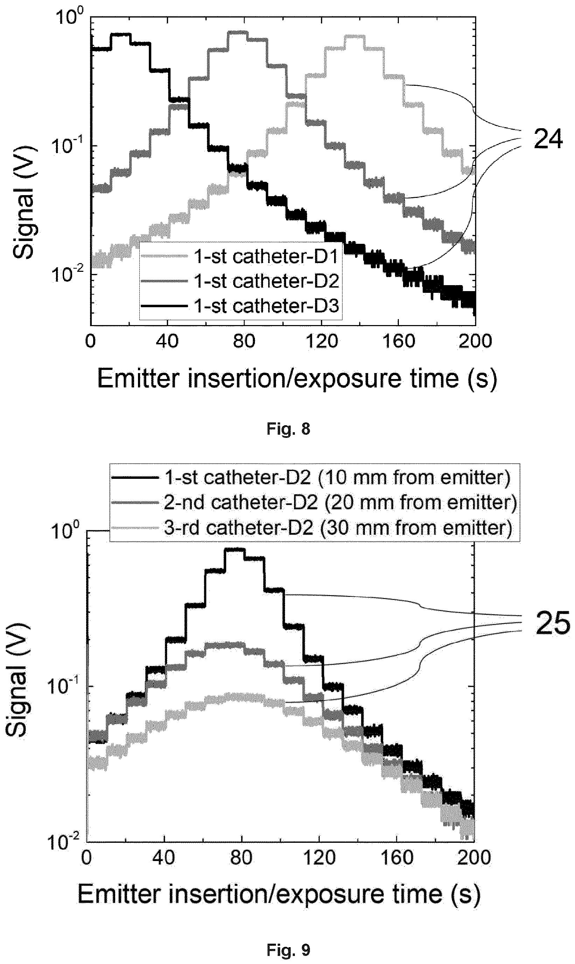

**Fig. 8.**   illustrates the 3D-scanner (2) calibration histograms (24) obtained by varying in discrete steps the position of the $^{192}$Ir radioisotope emitter (5) within a single *O*-needle-type catheter for different (scintillating (3) and diode (4)) sensors D1, D2, D3, allocated in the same (1-st) **C**-catheter;

**Fig. 9.**   illustrates the 3D-scanner (2) calibration histograms (25) recorded from the same sensors (D2) allocated within three different *C*-needle-type catheters (6) at different distances from the emitter (5) while varying in discrete steps the position of the $^{192}$Ir radioisotope emitter (5) in a single *O*-catheter (14);

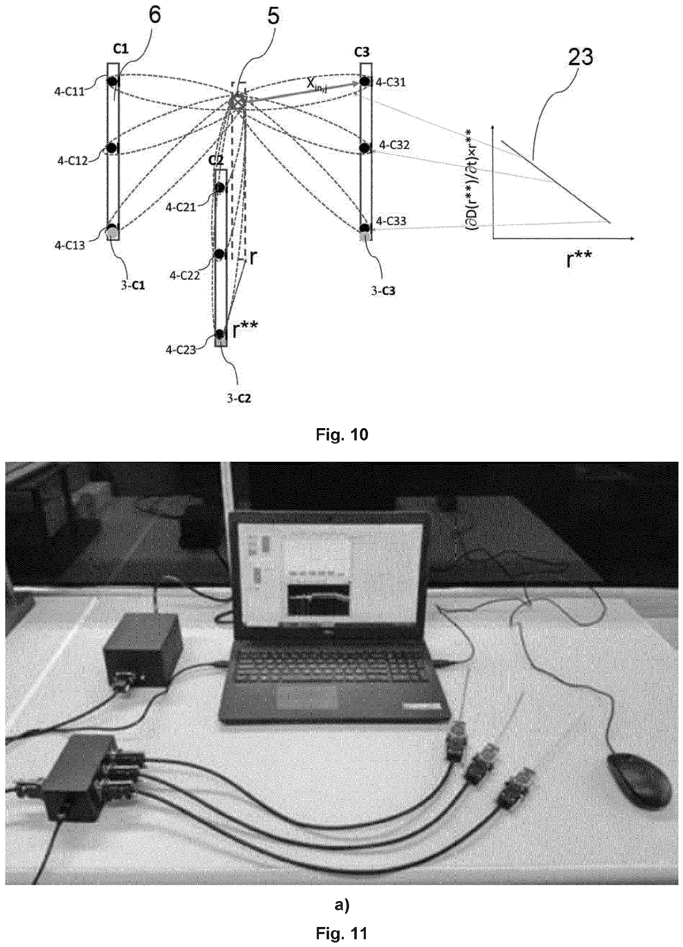

**Fig. 10.**   illustrates an arrangement of synchronous 3D-positioning and dosimetry system which is the 3D-scanner (2) comprising scintillator-sensors (3, 3-C1, 3-C2, 3-C3) and dosimeters (4-C11, 4-C12, 4-C13, 4-C21, 4-C22, 4-C23, 4-C31, 4-C32, 4-C33), installed within three needle-type scaling catheters (6) (*C1, C2, C3*) those positioned within the phantom (1) for brachytherapy planning and verification;

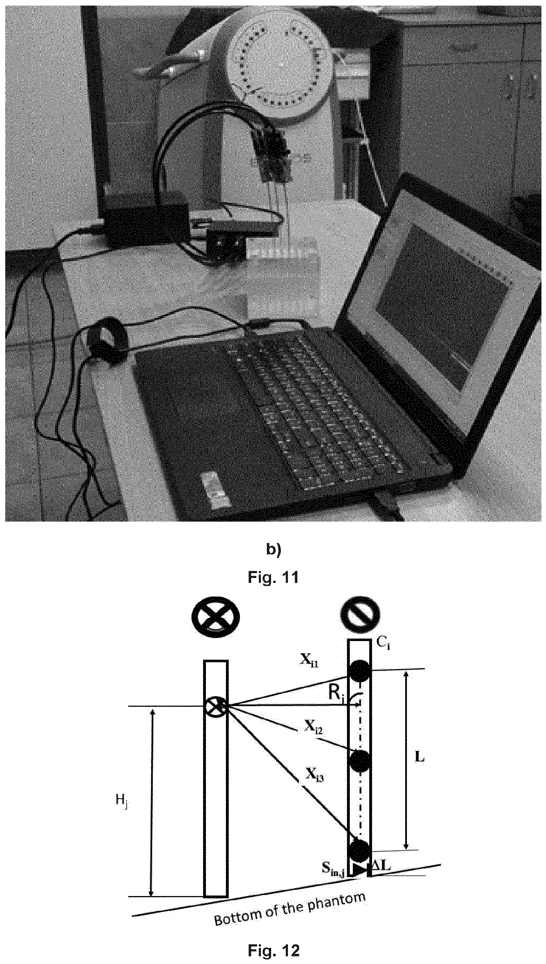

**Fig. 11.**   illustrates the photo-pictures of the tentative assemblies of the brachytherapy positioning and dosimetry system (2), where **(a)** the 3D-scanner catheters connected to a computer, and (b) the 3D-scanner catheters (6) inserted into the phantom (1) and afterloader (13) with radionuclide emitter (5);

**Fig. 12.**   represents the principle of estimating the distance $R_j$ of the radionuclide emitter (5) (within a calibration catheter *O*) relative to a specific probe (*C*i) having 3 sensors (*n*= 1, 2, 3) at spatial locations $X_{i1}, X_{i2}, X_{i3}$ (where *i*= 1, 2, 3 is the number of probes), and the height coordinate $H_j$ of the emitter (5) (at its horizontal plane *j*) measured vertically from the bottom of the phantom (1);

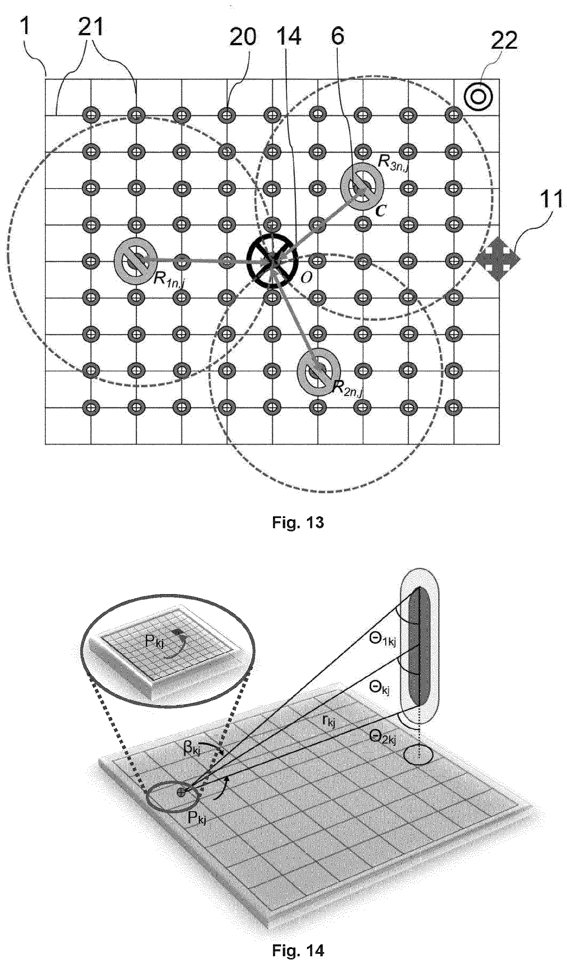

**Fig. 13.**   represents graphically the principle of determining instantaneous coordinates ($R_{1n,j}, R_{2n,j}, R_{3n,j}$) of the radionuclide (5) relative to the definite slice *j* using an intersection point *O* of 3 circles centred at definite probes (*i*) and sensors (*n*) located at distances $R_{in,j}$, measured relative to a position of the radiation source. Each section plane *j* is constructed using the meshes of the coaxial circles, those surrounding each of the cylindrical probes (*i*). $R_{in,j}$ is a distance from the definite probe axis to a point under examination;

**Fig. 14.**   represents corrections based on TG-43 formalism, applied for the enhanced precision in evaluation of the irradiation flux/dose, dependent on the polar angle $\theta$ within the cylindrical coordinate system, where $\beta$ is the angle subtended (defined) by the tips of an elongated radionuclide emitter (5) relative to the point *P* under consideration.

DRAWINGS - Reference Numerals

**1**   brachytherapy phantom, for replacing patient's tumour site during the brachytherapy plan verification stage;

**2**   3D-scanner for brachytherapy, comprising a system of sensor probes for synchronous 3D-positioning of radionuclide emitters and spatial dosimetry;

**3**   internal scintillator-sensors operating as optical beacons in the 3D-scanner system (2);

**4**   dosimeter-sensors, allocated in the probes together with the scintillator-sensors (3);

**5**   radionuclide emitter;

**6**   needle-type scaling catheters (at least, three catheters for the 3D-scanner system);

**7**   fiberscopes for transferring scintillator-sensor signals to optical/electrical signal digital converters;

**8**   optical/electrical-to-digital signal converters;

**9**   digital data transmission means from the signal converters (8) to a controlling computer, for further processing of the acquired data;

**10**  base-plate comprising a mesh-work with grid-shaped coordinate scaling, and with catheter installation grooves in it;

**11**  label (labels) for matching the base-plate (10) with patient's body or with the brachytherapy phantom (1), for precise fixing of 3D-scanner (2) probes therein;

**12**  applicator of a radionuclide emitter (5);

**13**  container or afterloader for housing the brachytherapy radionuclide (5);

**14**  installation groove with the catheter containing the radionuclide emitter inside;

**15**  imaging means by computer tomography (CT) or ultrasound scanning (US);

**16**  insulating ribbon substrate, installed within the needle-type catheter (6);

**17**  electrical wires for transferring the sensor electrical signals to digital converters;

**18**  electrical-to-digital converters;

**19**  optical-to-digital converters;

**20**  catheter (6) installation grooves in the base-plate (10) and phantom (1) body, the grooves spaced by the mesh-work coordinates on the base-plate;

**21**  mesh-work coordinate system on the base plate (10);

**22**  remote-position-verification-device (RPVD), such as LiDAR (laser imaging, detection, and ranging), ultrasound or laser distance meter, installed within the base-plate, and used for additional binding of the base-plate (10) to the label (11), marked e.g., on patient's body;

**23**  calibration characteristics of dosimeter signal as a function of distance from the $^{192}$Ir radionuclide emitter when using different type catheters (*C* and *O*) and phantom environments;

**24**  calibration histogram obtained by varying position of the $^{192}$Ir radioisotope emitter within single (O) needle-type catheter for a different type (scintillating and diode) sensors (3 and 4);

**25**  calibration histograms recorded varying the position of the $^{192}$Ir radioisotope emitter in series within various needle-type catheters.

DETAILED DESCRIPTION OF INVENTION

The present invention comprises a unified brachytherapy planning and verification complex comprising at least:

- A system (2) for brachytherapy synchronous 3D-positioning and dosimetry,
- A phantom (1) for brachytherapy procedure planning and verification;
- A method for 3D-positioning of brachytherapy radionuclide emitters (5), at least, in the phantom (1);
- A method for estimating brachytherapy irradiation spatial dose in 3D-coordinates (3D-dosimetry).

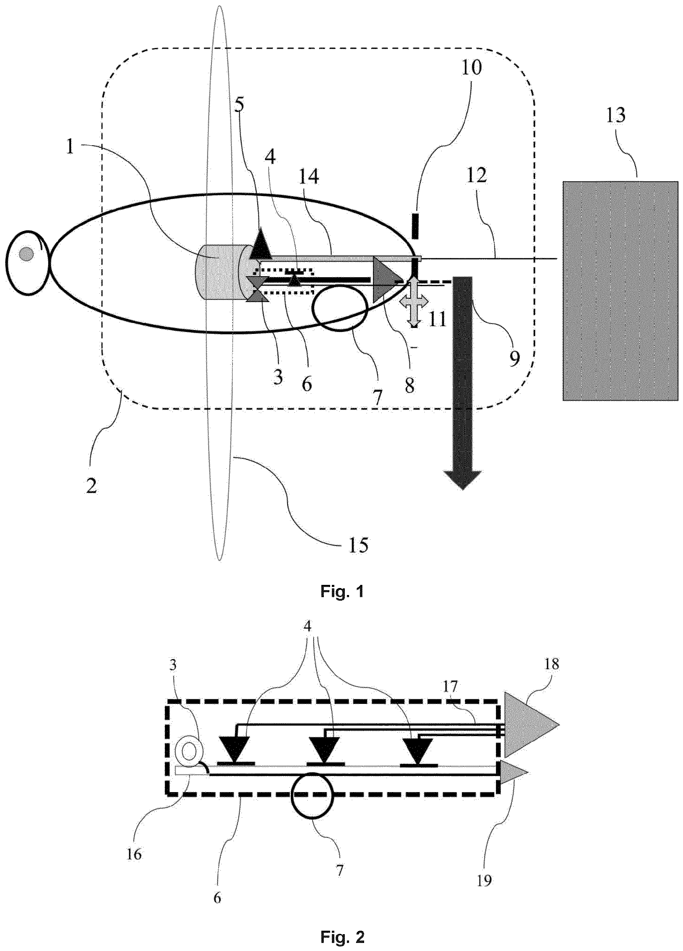

**System for brachytherapy radiation emitter spatial positioning and dosimetry.** The system is sketched in Figure 1. The system (2) of brachytherapy synchronous spatial positioning and dosimetry comprises a synchronous 3D-scanner composed of an arrangement of internal scintillator-sensors (3) operating as optical beacons. These scintillating sensors (3) are installed within, at least, three needle-type scaling catheters (6). These needle-type scaling catheters (6) can be installed and positioned within:

- either therapeutic patient's organ (1), the contour or boundary of which is simultaneously imaged by CT or US means (15),

- or the phantom (1) for brachytherapy procedure planning and verification;

The coordinates of the 3D-scanner (3-9) are tightly linked to a base-plate (10). The base-plate (10) comprises a mesh-work and grid-shaped 2D-coordinate scale indicators (21) linked to a plurality of catheter-installation grooves (20) wherein the needle-type catheters (6) are installed and thus also linked to a base-plate (10). The base-plate (10) further has a label (11) for linking its coordinates in relation to spatial 3D-coordinates of the phantom (1) or patient's body and tumour site. The 3D-scanner system (2) is linked to the phantom (1) or patient's body when the label (11) of the base-plate (10) is linked in relation to another label (11) marked on the phantom (1) or patient's body.

The base-plate (10) is coupled to the brachytherapy phantom (1) within the brachytherpy planning procedure, and the phantom (1), preferably, also comprises a plurality of catheter-installation grooves (20), where those grooves precisely match the grooves (20) of the base-plate (10) when the base-plate (10) and phantom (1) become linked by their coordinates. The needle-type catheters (6) installed into the grooves (20) of the base-plate (10), also extend into and are positioned within the phantom (1) and its 3D-coordinates.

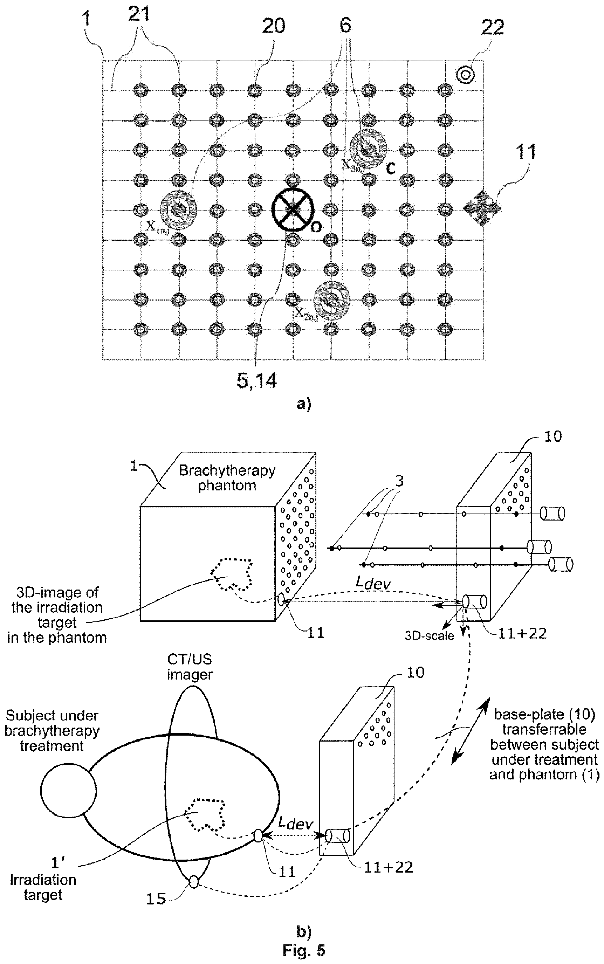

The linking of coordinates (21) of the base-plate (10) to the coordinates of the phantom (1) or patient's body may continuously (dynamically) be supported using remote-position-verification-device (RPVD) (22). The RPVD (22) may be LiDAR, ultrasound, or laser distance meters, installed within the base-plate (10). The RPVD (22) can support bound to a label on the patient's body, which is related to the tumour site and boundary contour. Such dynamic arrangement of RPVD (22) and base-plate (10) binding is important mainly for independent control of the base-plate (10) (and consequently of the 3D-scanner) position relative to the irradiation target (tumour contour) (1') within the patient's body. The coordinates' linking and control procedures (algorithms) are additionally implemented, ensuring instantaneous measurements of a distance from the patient's body under test (or treatment), to precisely determine the appeared position deviations $L_{dev}$ (Figure 5b) inevitable for the advanced dose calculations.

Operation of the 3D-scanner (3-9) with its scintillating sensors (3) is based on scintillation signal transfer from sensors (3) by fiberscopes (7) to optical/electrical-digital converters (8) and further transmitted by digital means (9) to the control computer inputs. The scintillator-sensor (3) signals are also used for additional positioning of the computer tomography (CT) imager X-ray source (15). This facilitates binding of the CT X-ray source coordinates to the base-plate (10) position and scale (21) and, therefore, linking the 3D-scanner's (2) coordinates to the tumour site and its contour (1) within the patient's body.

The 3D-scanner (2) is combined with radiation dosimeters (4), made of semiconductor device-type radiation sensors, and installed within the same scaling needle-type catheters (6) together with the scintillator-sensors (3). The triangulation technique (Fig. 5) is analogously applied for the signals from radiation sensors (4), and combined with the scaling performed using scintillating sensors (3, 3-9) of the 3D-scanner (Figures 1 and 10), to reach the enhanced precision of spatial positioning the radiation source (5) in time-space-resolved coordinates.

Other essential elements of the system and their features are mentioned in more detail in the "Description of Drawings" for Figure 2, Figure 3, Figure 4, Figure 5a, and the corresponding descriptions of elements in the "Drawings-Reference numerals" chapter.

The present brachytherapy positioning and dosimetry system (2) has the following advanced features, with respect to other known by state-of-art "real-time treatment dose verification system for afterloader brachytherapy systems":

- the present system is a 3D-scanner (2) made of scintillator-sensors (3) operating as temporal and position beacons installed within needle-type scaling and dosimetry catheters (6). Those are considered more efficient when being used instead of complicated external positioning systems known from scientific and patent literature and based on either additional electromagnetic tracking based on detection of magnetic fields, or X-ray-based flat-panel imagers, or computer tomography (CT)-based 3D-imaging systems, or combined positioning and dosimetry extended matrix systems;
- the 3D synchronous position-dosimetry scanner (2) employed in the present system, is made of a rigid system of three internal scintillator-sensors (3) and dosimeter-sensor (4) catheters (6), whereby provides a fast and optimal triangulation system for the tight binding of the 3D-scanner (2) to the scaling-calibrated base-plate (10) with a grid-shaped coordinate scale indicator (21) and the label (11) on the phantom (1) or patient's body;
- the 2D-coordinate scale (21) of the base-plate (10) is precisely bound to the patient's body or a phantom (1) using RPVD (22), such as LiDAR, ultrasound or laser distance meters, installed within the base-plate (10), which can be additionally bound to a label (11) on patient's body. The coordinate linking and control procedures additionally ensure the instantaneous measurements of a distance from the patient's body under test (or under treatment), to precisely determine the appeared position deviations $L_{dev}$ (Figure 5b) inevitable, due to patient's spontaneous movements, for the advanced dose calculations;
- for the brachytherapy procedure planning and verification, the 3D-phantom (1) is bound to the scaling-calibration base-plate (10) with a grid-shaped coordinate scale indicator (21) and patient's body label (11) containing a plurality of 3D-phantom installation grooves (20), in which a triangle (Figure 5a) of needle-type catheters (6) forming a 3D-position-dosimetry scanner (2), are initially installed, for initial calibration of the positioning system (2) where an initial calibration procedure is performed using the central catheter (14) filled with the same radioactive-isotope emitter (5), which is further employed for verification and treatment procedures, being provided from the brachytherapy afterloader (13) by the special applicator (12);

- brachytherapy verification procedures can be performed without using ultrasound or CT imaging instrumentation, when using a 3D-brachytherapy phantom (1) which is calibrated and bound, through the label (11) on the patient's body, to known tumour site (1') coordinates and boundary contour being related to a corresponding 3D-space inside the brachytherapy phantom (1);
- scintillation beacons (3) within double-response (scintillation and electrical) sensor probe (6) can also be employed for radiation-damage-control of the semiconductor dosimeters (4) inside the catheters (6) of the positioning and dosimetry scanner (2).

**Phantom apparatus.** The brachytherapy phantom apparatus (1) is intended to replace the patient's tumour site (1') during the stage of brachytherapy procedure plan verification. The phantom (1) is employed being coupled to the 3D-scanner system's base-plate (10). Coupling of the phantom (1) to the base plate (10) is precise due to matching by the label (11), implemented as surface cutouts, and also can further be continuously supported by RPVD (22) means, compensating in the real-time the mutual spatial displacements between the base-plate (10) and the phantom (1).

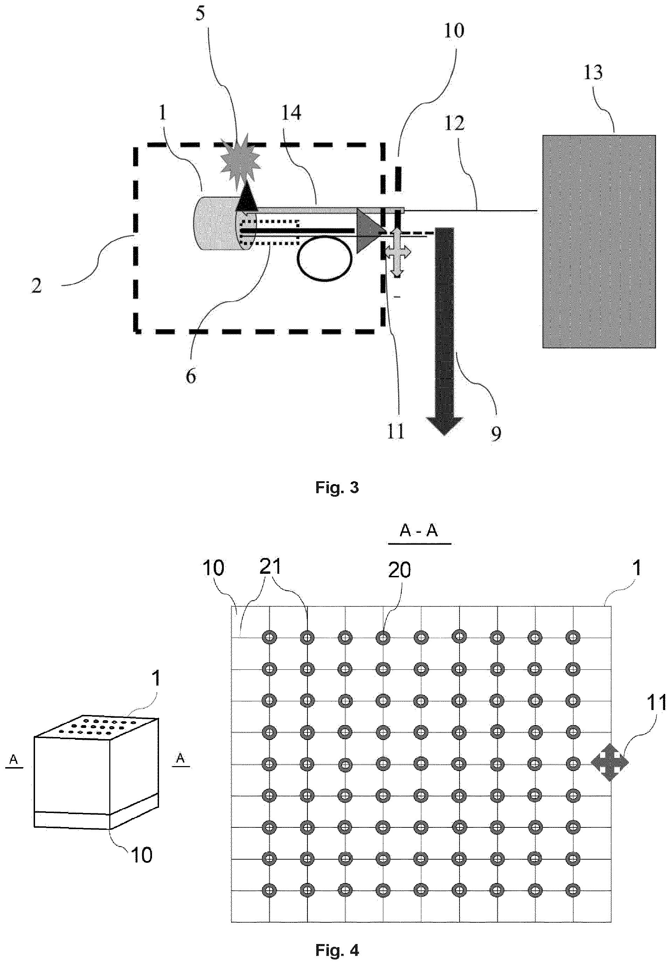

In a preferred embodiment (in Figures 6a-b), the phantom apparatus (1) is a cube-shaped body comprising a plurality of grooves (20) for installing brachytherapy catheters (6). These grooves (20) precisely match the coordinate mesh-work (21) on the base plate (10) and the counterpart installation grooves (20) at the base-plate (10). Therefore, the catheters (6) can be easily installed and quickly relocated among a plurality of 3D-mesh spatial points within the phantom (1) thus facilitating rapid and easy verification of brachytherapy procedure in an almost-real environment. Alternatively, the phantom (1) can be a cylinder-shaped body (Figures 1 and 3), which also provides easy and precise matching to the flat base-plate (10).

The cube-shaped phantom (1) comprises its 3D-coordinate-system mesh-work (21), where brachytherapy installation grooves (20) of the phantom (1) are precisely matched to this mesh-work (21). The phantom (1), in the case it is coupled to the base-plate (10) and the labels (11) matched, comprises the bound 3D-coordinate system with the base-plate (10), where the base-plate (10) coordinates become the reference coordinates also to the cube-shaped phantom (1).

The phantom (1), preferably, is made from materials, the density and radiation attenuation thereof is adjusted as close to the patient's body tissues under treatment. For example (Figure 6b), the phantom (1) can be produced from transparent acryl resembling by density body tissues, and it is transparent thus allowing visual inspection of the brachytherapy procedure emulated within it.

The phantom apparatus (1) having the above-disclosed features, with no restrictions, can be made of different volumes, sizes, shapes, materials, or compositions thereof, for the closest resemblance to body tissues, thus allowing more precise planning and verification of brachytherapy procedure.

**Matching of spatial coordinates.** This invention comprises a system of objects which are spatially separate 3D-objects but for efficient brachytherapy and spatial dosimetry, they have to be precisely aligned by their 3D-coordinate systems into a common system of 3D-coordinates. Thereby, each of objects has means for matching and positioning it in the common system of coordinates. Those are the reference coordinates of the base-plate (10), scales (21) of coordinate systems on objects, multiple labels (11) on objects for matching, RPVD (22) means to support and compensate dynamic deviations in real-time, means to specify exact coordinates of hidden objects (e.g., needle-type-catheters (6) installed into a body tissue under treatment) detectable by CT or US imagers (15), optical transparency of the phantom (1). When transferring the brachytherapy system (2) from the phantom to the subject under treatment, this common system of spatial 3D-coordinates is ensured by these means, as presented in Figure 5b.

**Positioning and dosimetry method.** This is a self-consistent method for brachytherapy procedure planning and verification on the phantom (1), before the treatment procedure on the patient's cancer tumour site (1'). The method has the following advanced features, with respect to other known by state-of-art "real-time treatment dose verification system for afterloader brachytherapy systems":

- the radioactive emitter (5) positioning method is self-consistent with instrumentation for the treatment irradiation procedures and avoids additional X-ray sources, such as CT imager, which may be detrimental for a patient;
- the method is based on the superposition of the patient's body label (11) with the scaling-calibration base-plate (10), containing a grid-shaped coordinate scale indicator (21) and the phantom (1) 3D-coordinates, and coincidence procedure is initially performed by using the central catheter (14) filled with the radioactive-isotope emitter (4);
- the method of simultaneous positioning and dosimetry is based on triangulation technique using a rigid triangle of the initially calibrated synchronous 3D-scanner and solving the optimization task using the stripe of 3 dosimeters (4) for rapid calculations in optimization solutions, instead of triangulation by optimization procedures made among multiple dosimeters (4) within prototype patent, where clear optimization is hardly achieved;

- linking of the base-plate (10) to the patient's body by RPVD (22) means, to ensure additionally a detection of the 3D-scanner's position from the patient's body under test (or treatment), to precisely determine the appeared position deviations $L_{dev}$ (Figure 5b) inevitable for the advanced dose calculations;
- the time-resolved, dynamic positioning procedures can be implemented by measuring transients of the analogical signals generated by the fast double-response scintillation-photoconductivity detectors, where position/distance equivalent delays are controlled.

**Positioning of radionuclide emitter.** The synchronous 3D-position-dosimetry scanner (2) is assembled (in Figure 10) to make a rigid triangulation system of at least three internal scintillator-sensor (3) and dosimeter-sensor (4) catheters (6), bound to a scaling-calibration base-plate (10) with a grid-shaped coordinate scale indicator (21), positioned on the 3D-phantom (1). The position on strip array of each scintillator-sensor (3) in every dosimetry catheter (6) of the 3D-scanner (2), acting as a temporal and position beacon, is determined and bound to the coordinate scale of the base-plate (10). The coordinate scale of the base-plate (10) is precisely bound to the patient's body or a phantom (1) using a remote-position-verification-device (RPVD) means (22), such as LiDAR (laser imaging, detection, and ranging), ultrasound, or laser distance meters, installed within base-plate (10) (Figure 5a), which can be additionally bound to a label (11) on patient's body. Such an arrangement of RPVD (22) and base-plate (10) bind is employed for independent control of the base-plate (10) position (and consequently of the 3D-scanner (2) position) relative to irradiation target (tumour) (1') within the patient's body. This also ensures the tight correlation between the coordinate scale of the base-plate (10) and dosimeters (4). The 3D-scanner (2) is composed, installed, and related to the base-plate (10) coordinate system. The central irradiation catheter (14) is installed, the radionuclide emitter (5) is shortly inserted into this catheter (14) from the afterloader (13) through a fixed applicator (12), the signals of scintillator- and dosimetry- sensors are registered by making the initial map (matrix) of temporal orientation-position-dose rate data. On the base-plate (10), either patient or phantom (1) labels (11) are superimposed. The data of a tumour site contour, bound with phantom (1) cube coordinates are loaded into the memory of the central computer when separate tumour site imaging is performed by using ultrasound or X-ray CT instruments (15). The signals of scintillator-sensors (3) and dosimeters (4) of the 3D-scanner (2) are additionally registered and superimposed with base-plate (10) and phantom (1) coordinates, using X-ray excitation sources within CT instrument (15) when tumour site contouring is synchronously performed (by CT) with initial calibration of the present brachytherapy positioning and dosimetry system. The fixed set of data such as $S_K$ (the Kerma factor evaluated in the air), $\Lambda$ (the dose rate constant), $G(r, \Theta)$ (the geometrical-factor function of the employed radionuclide), g(r) (the radial dose function), and $F(r, \Theta)$ (the anisotropy correction factor related to the angular dependent characteristic of the specific emitter activity) is initially provided for evaluating of the dose rate $\partial D(r, \Theta)/\partial t$, emitted by the definite radionuclide,-according to supplier information. Also, the calibrated characteristics (such as illustrated in Figures 7, 8, 9) of the amplitudes of each sensor (3, 4) response as a function of the distance $r^{**}$ between the radionuclide emitter (5) and the sensor (3, 4) (mounted within catheter cylinder) are measured and tabulated in advance.

The radionuclide emitter (5) is then inserted into the central radiation **O**-catheter (14), as illustrated in Figure 5a, installed within the patient's body or brachytherapy phantom (1), to compose the centred (at O) coordinate system, where the shortly inserted radionuclide emitter (1) serves for calibrating the initial positions of the probes (6) containing sensors ($n$=1, 2, 3) located at the distances $X_{i1,j}$, $X_{i2,j}$, $X_{i3,j}$ from a trial radionuclide emitter (5). Here, $i=1, 2, 3$ is the index of the probes. The spatial coordinates (those coincide with distances $X_{i1,j}$, $X_{i2,j}$, $X_{i3,j}$ for the system of catheter displacement centered at O) are determined using these distances from radionuclide to the definite sensor ($X_{i1,j}$, $X_{i2,j}$, $X_{i3,j}$) within the specific probe (i) and displaced along with the active probe of length ($L$), as illustrated in Figures 5a and 12. Values of distances $X_{i1,j}$, $X_{i2,j}$, $X_{i3,j}$ are extracted using the sensor (3, 4) response amplitudes dependent on distance $r^{**}$, registered under sensors (3, 4) excited by shortly inserted radionuclide emitter (5), and obtained by calibration measurements (the initial calibration signal histograms are illustrated in Figure 7, Figure 8, and Figure 9).

**Spatial dosimetry.** The parameters are extracted using optimization procedures for all the sensors (3, 4) involved within position-dosimetry 3D-scanner (2). The dose (D) is evaluated using the measured dose rate ($\partial D(r)/\partial t$) signals as [1]:

$$\dot{D}(r, \theta) = S_k \cdot \Lambda \cdot \frac{G(r, \theta)}{G(r_0, \theta_0)} \cdot g(r) \cdot F(r, \theta), \qquad (1)$$

where, $G(r_0, \Theta_0)$ is the initial geometrical-factor function for a single sensor (3, 4). The dose values $D(r_t, t_e)$ within the dosimetry map is obtained from the dose rate data using a dwelling time ($t_E$) of irradiation as [1]:

$$D(r_t, t_e) = [\partial D(r)/\partial t] \times t_E. \qquad (2)$$

The distance-position-dose values are obtained from the calibrated characteristics of the product $D(r, t_e) \times r^{**}$ being a linear function of a distance between the nuclide emitter (5) and the sensor $r^{**}$. The shortest distance is evaluated replacing the generalized factor $r^{**}$ by actual $R_i$ one ascribed to each (*i*) probe. The position $S_i$ of the scintillator-sensor (3) within the strip array of scintillator-sensors is also related to the strip length $\Delta L=0$ (Figure 12) measured relative to a bottom of the phantom (1) and catheter (6).

The algorithm used to identify the localization of the radiation source (for instance, $X_{i1,j}$) within a phantom (1) is based on the determination of sensor (3, 4) response, dependent on irradiation flux/dose rate, which decreases with the enhancement of a distance $R_i$ (from the radiation source) as the reciprocal function of $R_i^2$ (i.e. being the $1/R_i^2$, type function) when single directional variations are considered. The coordinates of the radiation source (5) in a phantom (1) are determined as follows:

- The altitude of a triangle $R_i$ from the radiation source to the specific (i) probe (Figure 12), containing sensors (*n*=1, 2, 3) located at $X_{i1,j}$, $X_{i2,j}$, $X_{i3,j}$ from radionuclide (where *i*=1, 2, 3 is the number of the probes), is related to the spatial coordinates (distances from radionuclide to the definite sensors ($X_{i1,j}$, $X_{i2,j}$, $X_{i3,j}$) within the specific probe displaced along active probe length (*L*) and can be evaluated by analyzing triangle (sketched on Figure 12) with sides of length *L*, $X_{i1,j}$, $X_{i3,j}$, and the semi-perimeter $s = (L + X_{i1,j} + X_{i3,j})/2$. The length of the altitude can then be expressed using Heron's formulae

$$R_i = 2 \frac{\sqrt{s(s-L)(s-X_{i1,j})(s-X_{i3,j})}}{L}. \qquad (3)$$

The latter relation is derived by equating the triangle area expressions ascribed to the same triangle composed of (1/2)×base(*L*)×altitude($R_i$) and that described by the Heron's formula $\sqrt{s(s-l)(s-X_{i1,j})(s-X_{i3,j})}$ in terms of the lengths. The altitude length $R_i$ can also be evaluated by using the equivalent form of the equation (3):

$$R_i = \frac{\sqrt{(X_{i1,j}+X_{i3,j}+L)(-X_{i1,j}+X_{i3,j}+L)(X_{i1,j}-X_{i3,j}+L)(X_{i1,j}+X_{i3,j}-L)}}{2L}. \qquad (4)$$

- The height coordinate $H_j$ related to each sensor n within horizontal (slices) section planes *j*, measured from the bottom of a phantom and ascribed to the vertical position of the radiation source within 3-D phantom, is evaluated (according to the illustration shown in Figure 12) using the equation:

$$H_j = \sqrt{X_{i3,j}^2 - R_i^2} + \Delta L \qquad (5)$$

- The instantaneous 3D-coordinates of the radiation source (5) within the horizontal section planes *j* (Figure 13), can be evaluated by determining the intersection points *O* of all the (*i*) probes located (within the definite horizontal plane) at different distances $R_{1j}$, $R_{2j}$ and $R_{3j}$ (*i*=1,2,3) relative to an instantaneous source position (similar to the illustration in Figure 13). It should be obtained a solution of a set (for instance, by considering the set of sensors *n*=1) of equations:

$$\begin{cases} (X - X_1)^2 + (Y - Y_1)^2 = R_1^2 \\ (X - X_2)^2 + (Y - Y_2)^2 = R_2^2 \\ (X - X_3)^2 + (Y - Y_3)^2 = R_3^2 \end{cases} \qquad (6)$$

- Further, the 3D coordinate system including each horizontal plane *j* is constructed by making the meshes of the coaxial circles surrounding each (*i*) cylindrical probe and solving a set of equations:

$$\{(X - X_{in,j})^2 + (Y - Y_{in,j})^2 + (Z - Z_{in,j})^2 = R_i^2 \qquad (7)$$

- There, the first circle is centered at the axis of every cylindrical probe and is chosen to be such to include the nearest nodes of the phantom mesh within any set of planar coordinates (to cover the scaling-calibration base-plate with a grid-shaped coordinate scale (21) indicator and 3D-phantom (1)). The radius of the successive circles is subsequently increased by a definite step within the evaluation mesh according to the desirable precision (to construct the initial coordinate mesh of the discrete pitches). Thereby, the principal coordinates of the mesh are set by calibrating the distances among the probes (catheters $C_i$ equipped with sensors *n*).

- The algorithm of dose calculation is based on the two-dimensional formalism (equation (1)) described in 2D AAPM TG-43 [7]. The phantom horizontal plane cross-section *j* is usually divided into squares and each square is divided into segments (dose points) $P_{kj}$ (where k is the number of the segment, for instance of $10 \times 10$ extent) similar as in a fragment of the phantom in Figure 14. Each dose point is calculated independently using TG-43 formalism based on the functions of the radial distance $r^{**}_{kj}$ from the radiation source and a polar angle coordinate $\theta_{kj}$, in the cylindrical coordinate system, meanwhile, $\beta_{kj}$ is the angle subtended by the tips of a radionuclide with respect to the point under test $P_{kj}$. This algorithm allows to consider a dose distribution within the cross-sectional planes *j* (slices).

Corrections due to the time delay ($\Delta t$) measured relative to a peak of the transient response are expressed as $\Delta X = c \times \Delta t$ with c being the speed of light. On the other hand, additional corrections should also be made concerning the actual strip height and deviations of its position $\Delta L$ relative to the bottom of the phantom.

Deviations from the planned doses and positions can be estimated by comparing the maps obtained using the phantom and those registered in the patient's body.

**Brachytherapy plan verification.** The brachytherapy procedure plan verification stage is performed by a complex arrangement of the 3D-scanner (2), the phantom (1), and the methods of positioning and dosimetry within the phantom (1), furthermore, complying with TG-43 formalism.

The brachytherapy procedure plan verification stage comprises at least these steps:

- the 3D-scanner (2) with its needle-type probes (6) is inserted through the base-plate (10) and mesh-work (21), into the phantom (1), and linked to the binding label (11) on the phantom (1);
- the position of the base-plate (10) relative to the phantom (1), is continuously monitored using RPVD (22) means, and position deviations are compensated during estimations of spatial irradiation doses;
- empty needle-type catheters (14) coupled with the radionuclide emitter applicators (12) are inserted into the phantom (1) grooves (20),
- the empty catheters (14) are allocated in the phantom (1) according to the tumour site contour;
- the radionuclide emitter (5) is further applied in a series of the pre-planned positions of irradiation, and,
- simultaneously, the scintillator-sensor (3) signals recorded to determine the position of the radionuclide emitter (5) within the 3D-scanner's (2) range;
- synchronously, changes of the 3D-scanner (2) signals are recorded;
- synchronously, ratios of dosimeter (4) amplitudes are recorded and instantaneously analysed, for terminating the verification of the sufficient irradiation dose collected;
- the matrix of data of the radionuclide emitter (5) positions is determined using correlations of different dosimeter (4) signals, among dosimeters (4) installed within different 3D-scanner (2) catheters (6),
- using the TG-43 formalism, analysis of the dosimeter (4) amplitudes, proportional to recorded dose rates and related to calibration characteristics, is performed for instantaneous estimating of spatially cumulated doses.

Furthermore, the collected 3D-dosimetry map also can be used as a reference for multiple iterative planning and verification steps, and ultimately, for the brachytherapy treatment procedure.

A tentative setup of the brachytherapy system is illustrated in Figures 10a-b.

NON-PATENT LITERATURE

1. D. Baltas and N.Zamboglou. 2D and 3D planning in brachytherapy. New Technologies in Radiation Oncology (Editors: W. Schlegel, T. Bortfeld, A.-L.Grosu), Springer , pp 237-254, https://doi.org/10.1007/3-540-29999-8, Springer-Verlag Berlin Heidelberg 2006, ISBN 978-3-540-29999-8.

**2.** P.Pittet, P.Jalade, L.Gindraux, P.Guiral, R.Wang, J.-M.Galvan, G.-N.Lu. DoRGaN: development of quality assurance and quality control systems for high dose rate brachytherapy based on GaN dosimetry probes. IRBM 39 (2018) 279-290.

**3.** M.Carrara, D.Cutajar, S.Alnaghy, A.Espinoza, A.Romanyukha, S.Pressila, C.Tenconi, A.Cerrotta, C.Fallai, M.-Safavi-Naeini, M. Petasecca, A.Kejda, M. Lerch, S. Corde, M.Jackson, A.Howie, J.Bucci, A.B.Rosenfeld. Semiconductor real-time quality assurance dosimetry in brachytherapy. Brachytherapy, 17 (2018) 133-145.

**4.** J.Cherpak, IJ.E.Cygler, C.E, G.Perry. Real-time measurement of urethral dose and position during permanent seed implantation for prostate brachytherapy. Brachytherapy, 13 (2014) 169-177.

**5.** A. M. Franz, T.Haidegger, W.Birkfellner, K. Cleary, T. M. Peters, and L. Maier-Hein. Electromagnetic tracking in medicine-a review of technology, validation, and applications. IEEE TRANSACTIONS ON MEDICAL IMAGING, 33 (2014) 1702-1725.

**6.** K.Tanderup, S.Beddar, C.E.Andersen, G.Kertzscher, J.Cygler. In vivo dosimetry in brachytherapy. Med. Phys. 40 (2013) 070902-01 - 070902-15.

**7.** M.J.Rivard, B.M.Coursey, L.A.DeWerd, W.F.Hanson, M.S.Huq, G.S.Ibbott, M.G. Mitch, R.Nath, J.F.Williamson. Update of AAPM Task Group No. 43 Report: A revised AAPM protocol for brachytherapy dose calculations. Med. Phys. 31 (2004) 633-674.

**Claims**

1. A brachytherapy positioning and dosimetry system for brachytherapy plan verification and treatment procedures, comprising at least a radionuclide emitter (5) applied from a brachytherapy afterloader (13),
   the system further comprises:

   • a base plate (10) comprising a plurality of grooves (20), a mesh-work with a grid-shaped coordinate-scale (21) linked to the mesh-work, and to a label (11) marked on a brachytherapy phantom or patient's body;
   • a 3D-scanner (2) comprising an arrangement of multiple scintillator-sensors (3) operating as optical beacons installed within, at least, three needle-type catheters (6), those dedicated to being positioned either within a phantom for brachytherapy procedure plan verification, or within patient's body tissue under treatment, whereby said at least three needle-type catheters (6) are installed into grooves (20) of the base plate (10) by their triangular allocation in the coordinate scale (21) of the mesh-work;
   • fiberscopes (7) connecting said scintillator-sensors (3), for transferring scintillators' signals for further processing;
   • the 3D-scanner (2) further comprises semiconductor device-type radiation sensors (4) as irradiation dosimeters, installed within said needle-type catheters (6) together with the scintillator-sensors (3);
   • a control computer, configured at least to:

      o control operation of the 3D-scanner (2) with the radionuclide emitter (5),
      o acquire the 3D-scanner data,
      o process the acquired 3D-scanner data, by applying triangulation technique on the signals of the radiation sensors (4) and the scintillator sensors (3), to identify positioning of the radionuclide emitter (5) within time-space-resolved coordinates,
      o measure the accumulated irradiation dose 3D-map within the time-space-resolved coordinates of the 3D-scanner (2), based on the signals from the dosimeters (4),

2. The system according to claim 1, wherein the system further comprises a Computer tomography imager X-ray source (15) and the control computer is further configured to use the scintillator-sensor (3) signals for positioning the Computer Tomography imager X-ray source (15), to bind its coordinates to the base-plate (10) position and scale, and to link the 3D-scanner's (2) coordinates to patient's tumour site and its boundary contour;

3. The system according to claim 1, wherein the quantity and spatial allocation of the dosimeters (4) within the needle-type catheters (6) is optimized for the shortest data-acquisition time and the minimal volume covering the patient's tumour site.

4. The system according to claims 1 to 4, wherein the system further comprises a remote-position-verification-devices (22), such as LiDAR, ultrasound, or laser distance meters, installed within the base-plate (10), and binding the base-plate (10) to the label (11) as well the tumour target in patient's body, to determine the occurred position deviations, inevitably requiring to correct the advanced dose estimations.

5. The system according to claim 1, wherein the scintillator-sensors (3) comprise wide-bandgap semiconductors, and the radiation-dosimeters (4) comprise narrow-bandgap semiconductors, for optimized spectral, temporal, spatial resolution, and sensitivity parameters of the 3D-scanner (2).

6. The system according to claim 1, wherein the scintillator-sensors (3) and radiation-dosimeters (4) comprise double-response wide-bandgap semiconductor sensors and different signal transfer channels, for performing spectroscopic dosimetry for point-dose measurements, and for localizing the radionuclide emitter (5) based on analysis of the spectral-peak-ratios.

7. The system according to claim 1, further comprising a phantom apparatus (1) for brachytherapy plan verification, having a volumetric, preferably, cube-shaped or cylinder-shaped body, the phantom comprising

   - brachytherapy installation grooves (20) for installing the needle-type-catheters (6, 14), and a 3D-coordinate-mesh-work (21), matching precisely said brachytherapy installation grooves (20),
   - coordinate-matching label (11), or labels, or surface cutouts, for matching the phantom (1) coordinates to the base-plate (10) and the 3D-scanner (2) coordinates,
   - wherein the phantom (1) is made from materials, the density and irradiation absorption thereof is close to the body tissues under treatment,
   - wherein, preferably, the phantom is transparent within visible spectral range.

8. A method of positioning the radionuclide emitter (5) within the system of claim 7, wherein the initial binding of the 3D-scanner (2) coordinates is established to the base-plate (10) linked to said at least three needle-type catheters (6) comprising a triangular allocation in the coordinate-scale (21), in the geometric centre of which a tentative catheter (14) with the radionuclide emitter (5) is briefly inserted and signals of scintillator-sensors (3) and radiation-dosimeters (4) are recorded as the reference signals for the base-plate (10)-bound coordinates.

9. The method according to claim 8, wherein instantaneous position of the applied radionuclide emitter (5) by different applicators (12) from the afterloader (13) is determined, by applying spatial triangulation technique, from synchronous analysis of the time-delays between fast-response scintillator-sensors (3) and amplitude differences of radiation-dosimeters (4), being allocated within different needle-type brachytherapy catheters (6).

10. The method according to claims 8 and 9, wherein

   - analogous transient signals of scintillator-sensors (3) are recorded during brachytherapy plan verification,
   - the time-resolved diagrams of dosimeter (4) amplitudes are related to the radionuclide emitter (5) coordinate mesh evaluated from scintillator-sensors (3) transients are considered simultaneously,
   - and the matrix of radionuclide emitter (5) positions are estimated using different dosimeter (4) signal correlations among dosimeters (4) and scintillator sensors (3) in different catheters (16), to increase the precision of the radionuclide emitter (5) positioning in the time-resolved 3D-scanner (2) coordinates.

11. A method of 3D-spatial dosimetry, using the system of claim 7, and the radionuclide emitter positioning method of claims 8 to 10,

   - wherein the base-plate (10) with mesh-work (21) labels (11) is linked to the phantom (1), to obtain a dosimetric map for brachytherapy plan verification,
   - wherein the positioning method allocates the radionuclide emitter (5) into the planned spatial positions for brachytherapy plan verification,
   wherein the cumulative 3D-map of instantaneous spatial doses is measured within 3D-coordinates of the base-plate(10)-related 3D-scanner (2), wherein said instantaneous spatial doses are estimated based on

- 1/R$^2$ law of irradiation dose dependence on the spherical-radius-distance R from the radionuclide emitter (5),
- and the calibrated signals of the dosimetry sensors (4).

**12.** The method according to claim 11, wherein estimation of geometric coordinates of the radionuclide emitter (5) positions and dose values is based on optimization tasks, wherein to get fast regression and local extremums, the minimal amount of 3D-scanner (2) catheters (6) and dosimeters (4) is determined.

**13.** The method according to claims 11 and 12, wherein the 3D-dosimetric map is a time-resolved 3D-matrix, composed by independently formed measurements of the 3D-matrix of dosimeter (4) responses, where the dosimeter (4) response amplitude relation to the dose-rate of the radionuclide emitter (5) with instantaneous activity is calibrated for a fixed distance between the dosimetry detectors (4) and the radionuclide emitter (5) in perpendicular geometry, using the needle-type brachytherapy catheters (6) and the radionuclide emitter (5).

**14.** A method for a brachytherapy procedure plan verification, using the system of claim 7, and methods of claims 8 to 10 and claims 11 to 13, wherein the method, complying with the Task Group No. 43, TG-43, of American Association of Physicists in Medicine formalism, comprises at least steps of

• the 3D-scanner (2) with its needle-type probes (6) is inserted through the base-plate (10) and mesh-work (21), into the phantom (1), by triangular allocation in the coordinate scale of the mesh-work (21), and linked to the binding label (11) on the phantom (1);
• the position of the base-plate (10) relative to the phantom (1), is continuously monitored using remote-position-verification-devices (22), and position deviations are compensated during estimations of spatial irradiation doses;
• empty needle-type catheters (14) coupled with the radionuclide emitter applicators (12) are inserted into the phantom (1) grooves (20),
• the empty catheters (14) are allocated in the phantom (1) according to the tumour site contour;
• the radionuclide emitter (5) is further applied in a series of the pre-planned positions of irradiation, and,

  ○ simultaneously, the scintillator-sensor (3) signals are recorded, and spatial triangulation technique is applied, to determine the position of the radionuclide emitter (5) within the 3D-scanner's (2) coordinates range;
  ○ synchronously, changes of the 3D-scanner (2) signals are recorded;
  ○ synchronously, ratios of dosimeters' (4) amplitudes are recorded and instantaneously considered, for terminating the verification of the sufficient irradiation dose applied;

• the matrix of radionuclide emitter (5) positions' data is determined using correlations of different dosimeter (4) signals, among dosimeters (4) installed within different 3D-scanner (2) catheters (6),
• using the TG-43 formalism, analysis of the dosimeter (4) amplitudes, proportional to recorded dose rates and related to calibration characteristics, is performed for instantaneous estimating of spatially cumulated doses.

**Patentansprüche**

**1.** Brachytherapeutisches Positionierungs- und Dosimetriesystem zur Überprüfung der brachytherapeutischen Planung und für brachytherapeutische Behandlungsabläufe, umfassend wenigstens einen Radionuklidemitter (5), der von einem brachytherapeutischen Nachlader (13) angewandt wird, wobei das System ferner umfasst:

eine Grundplatte (10), die eine Vielzahl von Nuten (20) umfasst, wobei ein Maschenwerk mit einer gitterförmigen Koordinatenskala (21), die mit dem Maschenwerk und mit einer Markierung (11) verknüpft ist, an einem Brachytherapiephantom oder dem Körper eines Patienten angebracht ist;
einen 3D-Scanner (2), der eine Anordnung von mehreren Szintillatorsensoren (3) umfasst, die als optische Festpunktmarkierungen dienen, die im Inneren von wenigstens drei Kathetern (6) des Nadeltyps installiert sind, welche speziell dazu dienen, entweder in einem Phantom zur Überprüfung eines brachytherapeutischen Behandlungsplans oder in dem behandelten Körpergewebe des Patienten positioniert zu werden, wobei die wenigstens drei Katheter (6) des Nadeltyps durch ihre Dreiecksallokation in der Koordinatenskala (21) des Maschenwerks in Nuten (20) der Grundplatte (10) installiert sind;
Fibroskope (7), die die Szintillatorsensoren (3) zum Übertragen von Signalen der Szintillatoren zur weiteren Verarbeitung verbinden;
wobei der 3D-Scanner (2) ferner Strahlungssensoren (4) des Halbleiterbauteiltyps als Bestrahlungsdosimeter umfasst, die zusammen mit den Szintillatorsensoren (3) in den Kathetern (6) des Nadeltyps installiert sind; einen Steuerungscomputer, der wenigstens zu Folgendem konfiguriert ist:

Steuern des Betriebs des 3D-Scanners (2) mit dem Radionuklidemitter (5),
Erlangen der 3D-Scannerdaten,
Verarbeiten der erlangten 3D-Scannerdaten durch Anwenden einer Triangulationstechnik auf die Signale der Strahlungssensoren (4) und der Szintillatorsensoren (3), um die Positionierung des Radionuklidemitters (5) in raumzeitlich aufgelösten Koordinaten zu ermitteln,
Messen der akkumulierten Bestrahlungsdosis-3D-Abbildung innerhalb der raumzeitlich aufgelösten Koordinaten des 3D-Scanners (2) auf Grundlage der Signale von den Dosimetern (4).

2. System nach Anspruch 1, wobei das System ferner eine Computertomographiebildgebungsröntgenquelle (15) umfasst und der Steuerungscomputer ferner dazu konfiguriert ist, die Signale der Szintillatorsensoren (3) zum Positionieren der Computertomographiebildgebungsröntgenquelle (15) zu verwenden, um ihre Koordinaten an die Position und die Skala der Grundplatte (10) anzubinden und die Koordinaten des 3D-Scanners (2) mit der Tumorstelle des Patienten und dessen Begrenzungskontur zu verknüpfen.

3. System nach Anspruch 1, wobei die Anzahl und die räumliche Allokation der Dosimeter (4) innerhalb der Katheter (6) des Nadeltyps für die kürzeste Datenerlangungszeit und das minimale Volumen optimiert werden, das die Tumorstelle des Patienten abdeckt.

4. System nach einem der Ansprüche 1 bis 4, wobei das System ferner Fern-Positionsüberprüfungsvorrichtungen (22) umfasst, wie etwa LiDAR-, Ultraschall- oder Laserentfernungsmessgeräte, die innerhalb der Grundplatte (10) installiert sind und die Grundplatte (10) an die Markierung (11) sowie das Tumorziel im Körper des Patienten anbinden, um aufgetretene Positionsabweichungen zu bestimmen, die unvermeidlich eine Korrektur der entwickelten Dosisschätzungen erfordern.

5. System nach Anspruch 1, wobei für eine optimierte spektrale, zeitliche, räumliche Auflösung und Sensitivitätsparameter des 3D-Scanners (2) die Szintillatorsensoren (3) Halbleiter mit breitem Bandabstand umfassen und die Strahlungsdosimeter (4) Halbleiter mit schmalem Bandabstand umfassen.

6. System nach Anspruch 1, wobei die Szintillatorsensoren (3) und die Strahlungsdosimeter (4) zweifach ansprechende Halbleitersensoren mit breitem Bandabstand und unterschiedlichen Signalübertragungskanälen umfassen, um eine spektroskopische Dosimetrie zur Punktdosenmessung durchzuführen und den Radionuklidemitter (5) auf Grundlage der Analyse der Verhältnisse der spektralen Maxima zu orten.

7. System nach Anspruch 1, ferner umfassend eine Phantomeinrichtung (1) zur Überprüfung der brachytherapeutischen Planung, die einen volumetrischen, vorzugsweise würfelförmigen oder zylinderförmigen Körper aufweist, wobei das Phantom umfasst

- brachytherapeutische Installationsnuten (20) zum Installieren der Katheter (6, 14) des Nadeltyps und ein 3D-Koordinaten-Maschenwerk (21), das genau mit den brachytherapeutischen Installationsnuten (20) übereinstimmt,
- eine Markierung (11) oder Markierungen oder Oberflächenausnehmungen zur Koordinatenabgleichung, um die Koordinaten des Phantoms (1) mit den Koordinaten der Grundplatte (10) und des 3D-Scanners (2) abzugleichen,
- wobei das Phantom (1) aus Materialien hergestellt ist, deren Dichte und Strahlungsabsorption nah an den behandelten Körpergeweben liegt,
- wobei das Phantom vorzugsweise innerhalb des sichtbaren Spektralbereichs transparent ist.

8. Verfahren zum Positionieren des Radionuklidemitters (5) in dem System nach Anspruch 7, wobei das anfängliche Anbinden der Koordinaten des 3D-Scanners (2) an der Grundplatte (10) erfolgt, die mit den wenigstens drei Kathetern (6) des Nadeltyps verknüpft ist, die eine Dreiecksallokation in der Koordinatenskala (21) umfassen, in deren geometrischer Mitte ein Versuchskatheter (14) mit dem Radionuklidemitter (5) kurzzeitig eingeführt wird, und Signale der Szintillatorsensoren (3) und der Strahlungsdosimeter (4) als die Referenzsignale für die an die Grundplatte (10) gebundenen Koordinaten verwendet werden.

9. Verfahren nach Anspruch 8, wobei die momentane Position des angewandten Radionuklidemitters (5) durch unterschiedliche Applikatoren (12) von dem Nachlader (13) durch Anwenden einer räumlichen Triangulationstechnik aus synchroner Analyse der Verzögerungen zwischen schnell ansprechenden Szintillatorsensoren (3) und Amplitudendifferenzen der Strahlungsdosimeter (4) bestimmt wird, die innerhalb unterschiedlicher Brachytherapiekatheter (6) des Nadeltyps zugeordnet sind.

10. Verfahren nach den Ansprüchen 8 und 9, wobei

- analoge Transientensignale der Szintillatorsensoren (3) während der Überprüfung der brachytherapeutischen Planung aufgezeichnet werden,
- die zeitlich aufgelösten Diagramme der Amplituden der Dosimeter (4) mit dem Koordinatengitter des Radionuklidemitters (5) in Beziehung stehen, das aus den Transienten der Szintillatorsensoren (3) beurteilt wird, simultan betrachtet werden,
- und die Matrix der Positionen des Radionuklidemitters (5) in unterschiedlichen Kathetern (16) unter Verwendung unterschiedlicher Signalkorrelationen der Dosimeter (4) zwischen den Dosimetern (4) und den Szintillatorsensoren (3) geschätzt werden, um die Genauigkeit der Positionierung des Radionuklidemitters (5) in den zeitlich aufgelösten Koordinaten des 3D-Scanners (2) zu erhöhen.

11. Verfahren zur Dosimetrie im 3D-Raum unter Verwendung des Systems nach Anspruch 7, und des Radionuklidemitterpositionierungsverfahrens nach den Ansprüche 8 bis 10,

- wobei die Grundplatte (10) mit Markierungen (11) des Maschenwerks (21) mit dem Phantom (1) verknüpft wird, um eine dosimetrische Abbildung zur Überprüfung der brachytherapeutischen Planung zu erhalten,
- wobei das Positionierungsverfahren den Radionuklidemitter (5) zur Überprüfung der brachytherapeutischen Planung an den geplanten räumlichen Positionen zuordnet,
wobei die kumulative 3D-Abbildung momentaner räumlicher Dosen innerhalb von 3D-Koordinaten des mit der Grundplatte (10) in Beziehung gesetzten 3D-Scanners (2) gemessen wird, wobei die momentanen räumlichen Dosen geschätzt werden auf Grundlage von
- dem $1/R^2$-Gesetz der Bestrahlungsdosisabhängigkeit von dem Kugelradiusabstand R von dem Radionuklidemitter (5),
- und den kalibrierten Signalen von den Dosimetriesensoren (4).

12. Verfahren nach Anspruch 11, wobei die Schätzung der geometrischen Koordinaten der Positionen des Radionuklidemitters (5) und der Dosenwerte auf Optimierungsaufgaben basiert, wobei zum Erhalten einer schnelleren Regression und lokaler Extreme die minimale Menge an Kathetern (6) und Dosimetern (4) des 3D-Scanners (2) bestimmt wird.

13. Verfahren nach den Ansprüchen 11 und 12, wobei die dosimetrische 3D-Abbildung eine zeitlich aufgelöste 3D-Matrix ist, die durch unabhängig gebildete Messungen der 3D-Matrix der Antworten der Dosimeter (4) zusammengesetzt ist, wobei unter Verwendung der Brachytherapiekatheter (6) des Nadeltyps und des Radionuklidemitters (5) die Beziehung der Ansprechamplitude der Dosimeter (4) zu der Dosisrate des Radionuklidemitters (5) mit sofortiger Aktivität für einen festen Abstand zwischen den Dosimetriedetektoren (4) und dem Radionuklidemitter (5) in senkrechter Geometrie kalibriert wird.

14. Verfahren zur Überprüfung eines brachytherapeutischen Behandlungsplans unter Verwendung des Systems nach Anspruch 7 und der Verfahren nach den Ansprüchen 8 bis 10 und den Ansprüchen 11 bis 13, wobei das Verfahren in Einhaltung der formellen Vorgaben von Task Group No. 43, TG-43 der American Association of Physicists in Medicine wenigstens folgende Schritte umfasst

der 3D-Scanner (2) mit seinen Sonden (6) des Nadeltyps wird mittels Dreiecksallokation in der Koordinatenskala des Maschenwerks durch die Grundplatte (10) und das Maschenwerk (21) in das Phantom (1) eingeführt (21) und mit der Anbindungsmarkierung (11) an dem Phantom (1) verknüpft;
die Position der Grundplatte (10) relativ zu dem Phantom (1) wird unter Verwendung von Fern-Positionsüberprüfungsvorrichtungen (22) kontinuierlich überwacht und Positionsabweichungen werden im Verlauf von Schätzungen von räumlichen Bestrahlungsdosen kompensiert;
leere Katheter (14) des Nadeltyps, die an die Applikatoren (12) des Radionuklidemitters gekoppelt sind, werden in die Nuten (20) des Phantoms (1) eingeführt,
die leeren Katheter (14) werden in dem Phantom (1) gemäß der Kontur der Tumorstelle zugeordnet;
der Radionuklidemitter (5) wird weiter in einer Reihe der im Voraus geplanten Positionen der Bestrahlung angewandt und

simultan werden die Signale der Szintillatorsensoren (3) aufgezeichnet und es wird eine räumliche Triangulationstechnik angewandt, um die Position des Radionuklidemitters (5) im Koordinatenbereich des 3D-Scanners (2) zu bestimmen;
synchron werden Änderung der Signale des 3D-Scanners (2) aufgezeichnet;
synchron werden Verhältnisse der Amplituden der Dosimeter (4) aufgezeichnet und sofort berücksichtigt, um die Überprüfung der angewandten ausreichenden Bestrahlungsdosis zu beenden;

die Matrix der Daten der Positionen des Radionuklidemitters (5) wird unter Verwendung von Korrelationen der Signale unterschiedlicher Dosimeter (4) unter den Dosimetern (4) bestimmt, die in unterschiedlichen Kathetern (6) des 3D-Scanners (2) installiert sind,
unter Verwendung der formellen Vorgaben von TG-43 wird die Analyse der Amplituden der Dosimeter (4) proportional zu aufgezeichneten Dosisraten und in Beziehung mit Kalibrationscharakteristiken für die sofortige Schätzung räumlich kumulierter Dosen durchgeführt.

## Revendications

1. - Système de positionnement et de dosimétrie de curiethérapie pour des procédures de vérification de plan de curiethérapie et de traitement, comprenant au moins un émetteur de radionucléides (5) appliqué à partir d'un projecteur de source de curiethérapie (13), le système comprenant en outre :

   • une plaque de base (10) comprenant une pluralité de rainures (20), un maillage avec une échelle de coordonnées en forme de grille (21) liée au maillage, et à un traceur (11) marqué sur un fantôme de curiethérapie ou le corps d'un patient ;
   • un scanner 3D (2) comprenant un agencement de multiples capteurs de scintillateur (3) fonctionnant en tant que balises optiques installées au sein d'au moins trois cathéters de type aiguille (6), ceux-ci étant prévus pour être positionnés soit au sein d'un fantôme pour une vérification de plan de procédure de curiethérapie, soit au au sein d'un tissu corporel d'un patient en cours de traitement, moyennant quoi lesdits au moins trois cathéters de type aiguille (6) sont installés dans des rainures (20) de la plaque de base (10) par leur allocation triangulaire dans l'échelle de coordonnées (21) du maillage ;
   • des fibroscopes (7) connectant lesdits capteurs de scintillateur (3), pour transférer des signaux de scintillateurs pour un traitement ultérieur ;
   • le scanner 3D (2) comprend en outre des capteurs de rayonnement de type dispositif à semi-conducteur (4) en guise de dosimètres d'irradiation, installés au sein desdits cathéters de type aiguille (6) conjointement avec les capteurs de scintillateur (3) ;
   • un ordinateur de commande, configuré au moins pour :

      ◦ commander le fonctionnement du scanner 3D (2) avec l'émetteur de radionucléides (5),
      ◦ acquérir les données de scanner 3D,
      ◦ traiter les données de scanner 3D acquises, en appliquant une technique de triangulation sur les signaux des capteurs de rayonnement (4) et les capteurs de scintillateur (3), pour identifier un positionnement de l'émetteur de radionucléides (5) au sein de coordonnées résolues dans le temps et l'espace,
      ◦ mesurer la carte 3D de dose d'irradiation cumulée au sein des coordonnées résolues dans le temps et l'espace du scanner 3D (2), en fonction des signaux provenant des dosimètres (4),

2. - Système selon la revendication 1, le système comprenant en outre une source de rayons X d'imageur de tomographie par ordinateur (15) et l'ordinateur de commande étant configuré en outre pour utiliser les signaux du capteur de scintillateur (3) pour positionner la source de rayons X d'imageur de tomographie par ordinateur (15), pour relier ses coordonnées à la position et à l'échelle de la plaque de base (10), et pour relier les coordonnées du scanner 3D (2) au site tumoral du patient et à son contour de délimitation ;

3. - Système selon la revendication 1, dans lequel la quantité et l'allocation spatiale des dosimètres (4) au sein des cathéters de type aiguille (6) sont optimisées pour le temps d'acquisition de données le plus court et le volume minimal couvrant le site tumoral du patient.

4. - Système selon les revendications 1 à 4, le système comprenant en outre des dispositifs de vérification de position à distance (22), tels que LiDAR, ultrasons ou télémètres laser, installés au sein de la plaque de base (10), et reliant la plaque de base (10) au traceur (11) ainsi que la cible tumorale dans le corps du patient, pour déterminer des écarts de position qui surviennent, nécessitant inévitablement de corriger les estimations de dose avancées.

5. - Système selon la revendication 1, dans lequel les capteurs de scintillateur (3) comprennent des semi-conducteurs à large bande interdite, et les dosimètres de rayonnement (4) comprennent des semi-conducteurs à bande interdite étroite, pour une optimisation de la résolution spectrale, temporelle, spatiale, et des paramètres de sensibilité du scanner 3D (2).

6. - Système selon la revendication 1, dans lequel les capteurs de scintillateur (3) et les dosimètres de rayonnement (4) comprennent des capteurs à semi-conducteurs à large bande interdite à double réponse et différents canaux de transfert de signal, pour mettre en oeuvre une dosimétrie spectroscopique pour des mesures de doses ponctuelles, et pour localiser l'émetteur de radionucléides (5) en fonction d'une l'analyse des rapports de crête spectrale.

7. - Système selon la revendication 1, comprenant en outre un appareil fantôme (1) pour une vérification de plan de curiethérapie, ayant un corps volumétrique, de préférence en forme de cube ou en forme de cylindre, le fantôme comprenant

- des rainures d'installation de curiethérapie (20) pour l'installation des cathéters de type aiguille (6, 14), et d'un maillage de coordonnées 3D (21), correspondant précisément auxdites rainures d'installation de curiethérapie (20),
- un traceur (11), ou des traceurs, de mise en correspondance de coordonnées, ou des encoches de surface, pour la mise en correspondance des coordonnées du fantôme (1) avec la plaque de base (10) et les coordonnées du scanner 3D (2),
- le fantôme (1) étant fabriqué à partir de matériaux, dont la masse volumique et l'absorption d'irradiation sont proches des tissus corporels en traitement,
- de préférence, le fantôme étant transparent au sein de la gamme spectrale visible.

8. - Procédé de positionnement de l'émetteur de radionucléides (5) au sein du système selon la revendication 7, dans lequel la liaison initiale des coordonnées du scanner 3D (2) est établie sur la plaque de base (10) reliée auxdits au moins trois cathéters de type aiguille (6) comprenant une allocation triangulaire dans l'échelle de coordonnées (21), au centre géométrique duquel un cathéter provisoire (14) avec l'émetteur de radionucléides (5) est brièvement inséré et des signaux des capteurs de scintillateur (3) et des dosimètres de rayonnement (4) sont enregistrés en guise de signaux de référence pour les coordonnées reliées à la plaque de base (10).

9. - Procédé selon la revendication 8, dans lequel la position instantanée de l'émetteur de radionucléides appliqué (5) par différents applicateurs (12) provenant du projecteur de source (13) est déterminée, en appliquant une technique de triangulation spatiale, à partir d'une analyse synchrone des retards temporels entre des capteurs de scintillateur à réponse rapide (3) et des différences d'amplitude des dosimètres de rayonnement (4), étant alloués au sein de différents cathéters de curiethérapie de type aiguille (6).

10. - Procédé selon les revendications 8 et 9, dans lequel

- des signaux transitoires analogiques de capteurs de scintillateur (3) sont enregistrés pendant une vérification de plan de curiethérapie,
- les diagrammes à résolution temporelle d'amplitudes du dosimètre (4) se rapportent au maillage de coordonnées de l'émetteur de radionucléides (5) évalué, les régimes transitoires de capteurs de scintillateur (3) sont considérés simultanément,
- et la matrice de positions de l'émetteur de radionucléides (5) est estimée à l'aide de différentes corrélations de signal de dosimètre (4) parmi les dosimètres (4) et capteurs de scintillateur (3) dans différents cathéters (16), pour augmenter la précision de positionnement de l'émetteur de radionucléides (5) dans les coordonnées du scanner 3D (2) à résolution temporelle.

11. - Procédé de dosimétrie spatiale 3D, à l'aide du système selon la revendication 7, et procédé de positionnement d'émetteur de radionucléides selon les revendications 8 à 10,

- dans lequel la plaque de base (10) avec des traceurs (11) maillage (21) est reliée au fantôme (1), pour obtenir une carte dosimétrique pour une vérification de plan de curiethérapie,

- dans lequel le procédé de positionnement alloue l'émetteur de radionucléides (5) dans les positions spatiales planifiées pour une vérification de plan de curiethérapie,

dans lequel la carte 3D cumulative des doses spatiales instantanées est mesurée au sein des coordonnées 3D du scanner 3D (2) se rapportant à la plaque de base (10), lesdites doses spatiales instantanées étant estimées en fonction de

- la loi $1/R^2$ de dépendance de dose d'irradiation par rapport à la distance R de rayon sphérique de l'émetteur de radionucléides (5),
- et des signaux étalonnés des capteurs de dosimétrie (4).

12. - Procédé selon la revendication 11, dans lequel l'estimation de coordonnées géométriques des positions de l'émetteur de radionucléides (5) et des valeurs de dose est en fonction de tâches d'optimisation, dans lesquels pour obtenir une régression rapide et des valeurs extrêmes locales, la quantité minimale de cathéters (6) et dosimètres (4) du scanner 3D (2) est déterminée.

13. - Procédé selon les revendications 11 et 12, dans lequel la carte dosimétrique 3D est une matrice 3D à résolution temporelle, composée de mesures formées indépendamment de la matrice 3D de réponses du dosimètre (4), où la relation d'amplitude de réponse du dosimètre (4) au débit de dose de l'émetteur de radionucléides (5) avec une activité instantanée est étalonnée pour une distance fixe entre les détecteurs de dosimétrie (4) et l'émetteur de radionucléides (5) en géométrie perpendiculaire, à l'aide des cathéters de curiethérapie de type aiguille (6) et de l'émetteur de radionucléides (5).

14. - Procédé pour une vérification de plan de procédure de curiethérapie, à l'aide du système selon la revendication 7, et procédés selon les revendications 8 à 10 et selon les revendications 11 à 13, le procédé, répondant au formalisme du Groupe de travail No. 43, TG-43, de l'American Association of Physicists in Medicine, comprenant au moins les étapes

• le scanner 3D (2) avec ses sondes de type aiguille (6) est inséré à travers la plaque de base (10) et un maillage (21), dans le fantôme (1), par allocation triangulaire dans l'échelle de coordonnées du maillage (21), et relié au traceur de liaison (11) sur le fantôme (1) ;
• la position de la plaque de base (10) par rapport au fantôme (1), est surveillée de façon continue à l'aide de dispositifs de vérification de position à distance (22), et des déviations de position sont compensées pendant des estimations de doses d'irradiation spatiales ;
• des cathéters de type aiguille vides (14) couplés aux applicateurs d'émetteur de radionucléides (12) sont insérés dans les rainures (20) du fantôme (1),
• les cathéters vides (14) sont alloués dans le fantôme (1) selon le contour de site tumoral ;
• l'émetteur de radionucléides (5) est en outre appliqué dans une série des positions pré-planifiées d'irradiation, et,

◦ simultanément, les signaux du capteur de scintillateur (3) sont enregistrés, et une technique de triangulation spatiale est appliquée, pour déterminer la position de l'émetteur de radionucléides (5) au sein de la plage de coordonnées du scanner 3D (2) ;
◦ de façon synchrone, des changements des signaux du scanner 3D (2) sont enregistrés ;
◦ de façon synchrone, des rapports des amplitudes des dosimètres (4) sont enregistrés et considérés instantanément, pour terminer la vérification de la dose d'irradiation suffisante appliquée ;

• la matrice des données de position de l'émetteur de radionucléides (5) est déterminée à l'aide de corrélations de signaux de différents dosimètres (4), parmi les dosimètres (4) installés au sein des différents cathéters (6) du scanner 3D (2),
• à l'aide du formalisme TG-43, une analyse des amplitudes du dosimètre (4), proportionnelle aux débits de dose enregistrés et se rapportant à des caractéristiques d'étalonnage, est mise en oeuvre pour une estimation instantanée de doses cumulées dans l'espace.

**Fig. 1**

**Fig. 2**

Fig. 3

A - A

Fig. 4

a)

b)

Fig. 5

a)

b)

Fig. 6

**Fig. 6 c**

**Fig. 7**

Fig. 8

Fig. 9

Fig. 10

a)

Fig. 11

b)

Fig. 11

Fig. 12

**Fig. 13**

**Fig. 14**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 100613244 B1
- KR 101752972 B1
- AU 2008100728 A4
- WO 2020246728 A

### Non-patent literature cited in the description

- 2D and 3D planning in brachytherapy. **D. BALTAS ; N.ZAMBOGLOU.** New Technologies in Radiation Oncology. Springer, 237-254
- **P.PITTET ; P.JALADE ; L.GINDRAUX ; P.GUIRAL ; R.WANG ; J.-M.GALVAN ; G.-N.LU.** DoRGaN: development of quality assurance and quality control systems for high dose rate brachytherapy based on GaN dosimetry probes. *IRBM,* 2018, vol. 39, 279-290
- **M.CARRARA ; D.CUTAJAR ; S.ALNAGHY ; A.ESPINOZA ; A.ROMANYUKHA ; S.PRESSILA ; C.TENCONI ; A.CERROTTA ; C.FALLAI ; M.-SAFAVI-NAEINI.** Semiconductor real-time quality assurance dosimetry in brachytherapy. *Brachytherapy,* 2018, vol. 17, 133-145
- **J.CHERPAK ; IJ.E.CYGLER, C.E ; G.PERRY.** Real-time measurement of urethral dose and position during permanent seed implantation for prostate brachytherapy. *Brachytherapy,* 2014, vol. 13, 169-177
- **A. M. FRANZ ; T.HAIDEGGER ; W.BIRKFELLNER ; K. CLEARY ; T. M. PETERS ; L. MAIER-HEIN.** Electromagnetic tracking in medicine-a review of technology, validation, and applications. *IEEE TRANSACTIONS ON MEDICAL IMAGING,* 2014, vol. 33, 1702-1725
- **K.TANDERUP ; S.BEDDAR ; C.E.ANDERSEN ; G.KERTZSCHER ; J.CYGLER.** In vivo dosimetry in brachytherapy. *Med. Phys,* 2013, vol. 40, 070902-01, 070902-15
- **M.J.RIVARD ; B.M.COURSEY ; L.A.DEWERD ; W.F.HANSON ; M.S.HUQ ; G.S.IBBOTT ; M.G. MITCH ; R.NATH ; J.F.WILLIAMSON.** Update of AAPM Task Group No. 43 Report: A revised AAPM protocol for brachytherapy dose calculations. *Med. Phys.,* 2004, vol. 31, 633-674